# FASCICULE DE BREVET EUROPEEN

(11) **EP 2 558 061 B1**
(45) Date de publication et mention de la délivrance du brevet: **05.09.2018**
(21) Numéro de dépôt: 11731417.9
(22) Date de dépôt: 14.04.2011
(51) Int. Cl.: A61K 8/9789, A61Q 19/00, A61Q 19/08

(54) **UTILISATION D'UN HYDROLYSAT PEPTIDIQUE DE POIS EN TANT QU'AGENT ACTIF HYDRATANT**
VERWENDUNG EINES PEPTIDHYDROLYSATS AUS BOHNEN ALS BEFEUCHTUNGSWIRKSTOFF
USE OF A PEPTIDE HYDROLYSATE OF PEA AS MOISTURIZING ACTIVE AGENT

(30) Priorité: 15.04.2010 FR 1001604
(43) Date de publication de la demande: 20.02.2013
(73) Titulaire: ISP Investments Inc., Wilmington, DE 19805 (US)
(72) Inventeur: DAL FARRA, Claude, Kerhonkson, New York 12446 (US); DOMLOGE, Nouha, F-06560 Valbonne (FR); BOTTO, Jean-Marie, F-06560 Valbonne (FR)
(74) Mandataire: Macquet, Christophe
(86) Numéro de dépôt international: PCT/FR2011/000219
(87) Numéro de publication internationale: WO 2011/128530

(56) Documents cités:
- EP-A2- 2 033 617
- WO-A1-86/02833
- WO-A1-02/055049
- WO-A1-2008/151890
- WO-A2-2008/015341
- FR-A1- 2 904 556
- JP-A- 9 025 225
- JP-A- 2008 100 929
- JP-A- 2008 100 930

## Description

### Domaine de l'invention

La présente invention se situe dans le domaine cosmétique, et pharmaceutique, et plus particulièrement dans le domaine de la dermatologie. L'invention concerne l'utilisation cosmétique d'un hydrolysat peptidique de pois (*Pisum sativum* L.) selon les revendications en tant qu'agent actif hydratant. L'invention concerne plus particulièrement l'utilisation cosmétique d'un hydrolysat peptidique de pois *(Pisum sativum* L.) pour améliorer l'hydratation constitutive de la peau, grâce à ses propriétés activatrices de l'expression des aquaporines, des glycosaminoglycanes et de la filaggrine.

L'invention concerne encore un procédé de traitement cosmétique destiné à restaurer le déséquilibre hydrique qui se manifeste lors du vieillissement cutané.

L'agent actif peut être utilisé seul ou en association avec d'autres agents actifs.

L'invention est également relative à l'utilisation de ce nouvel agent actif pour la réalisation d'une composition pharmaceutique, et notamment dermatologique, destinée à prévenir ou traiter les sécheresses pathologiques de la peau.

### Arrière-plan de l'invention

La peau est un organe vital composé de plusieurs couches (derme, couches prolifératives et *stratum corneum*) qui recouvre toute la surface du corps et assure essentiellement une fonction de barrière vis-à-vis du milieu extérieur. La qualité et le bon fonctionnement de la peau sont étroitement liés à la teneur en eau des différentes couches de l'épiderme et du derme. Ainsi, dans un épiderme normal, les couches prolifératives contiennent environ 70 % d'eau, alors que le *stratum corneum* en contient seulement 10 à 15 %.

L'hydratation de la peau est la résultante de trois facteurs : l'approvisionnement en eau à partir de la circulation des fluides physiologiques, la perte en eau vers le milieu extérieur et enfin la capacité des différents compartiments de la peau à fixer les molécules d'eau.

La régulation de l'approvisionnement en eau se fait par des hormones (aldostérone, hormones sexuelles), le pH ou les variations osmotiques. Les membranes cellulaires sont de nature hydrophobes et donc peu perméables à l'eau, mais il existe des canaux hydriques, sorte de pores facilitant le passage de l'eau et de certains solutés.

Les aquaporines sont une classe de protéines transmembranaires transporteurs d'eau et de petites molécules en solution. Les aquaporines de type 3, ou AQP3, sont présentes dans l'épiderme humain, et plus précisément dans les kératinocytes des couches prolifératives de l'épiderme (Sougrat R. et al., J. Invest. Dermatol., 2002). Elles sont capables de transporter l'eau et le glycérol, ce dernier jouant un rôle important dans la constitution du film hydrolipidique de surface ainsi que dans le maintien de la souplesse et des qualités sensorielles du *stratum corneum.* L'hydratation et la teneur en AQP3 des kératinocytes sont étroitement liées. Ainsi, l'augmentation d'AQP3 dans la peau provoque une meilleure hydratation de l'épiderme (Dumas M., J. Drugs Dermatol., Jun6, 2007). D'autre part, les aquaporines jouent un rôle dans la fonction barrière en régulant positivement l'établissement des liaisons et communications cellulaires de type tight junction (Kawedia J. et al., PNAS 104(9), 2007).

Les couches cornées (*stratum corneum*) forment une barrière essentielle pour limiter les pertes en eau. Dans les couches les plus profondes du *stratum corneum,* durant la formation des cornéocytes, la profilaggrine insoluble présente dans les kératinocytes granuleux se transforme en filaggrine et devient ainsi capable de se lier aux fibres de kératine pour former des microfibrilles. La filaggrine est également impliquée dans le maintien de la teneur en eau des couches cornées. Son devenir lors de la maturation des cornéocytes dépend du gradient d'eau dans le *stratum corneum.* Dans une peau normale, en situation de faible humidité ambiante, la filaggrine est hydrolysée et libère des substances hygroscopiques et solubles composées d'acides aminés et de dérivés d'acides aminés qui constituent une partie du « Natural Moisturizing Factor » ou NMF. Le NMF a la propriété de capter l'eau atmosphérique et de retenir l'eau du *stratum corneum,* pour maintenir la peau souple et flexible, et permettre les réactions enzymatiques nécessaires à l'évolution des cornéocytes vers l'étape ultime de la desquamation. Toutefois lors de certaines pathologies (ex. xérose, atopie) ou encore lorsque la peau est âgée, le NMF est en quantité insuffisante pour assurer ses fonctions hydratantes ou peut subir une dégradation ou traverser la membrane cellulaire et laisser échapper progressivement l'eau qui s'évapore dans le milieu extérieur.

Toutefois, les principales réserves en eau sont situées dans le derme, qui contient jusqu'à 80 % d'eau, dans le cas d'une peau jeune (ce taux diminue avec l'âge). L'eau du derme provient du plasma et est étroitement liée aux glycosaminoglycanes (GAG) et aux glycoprotéines de structure. La principale fonction des glycosaminoglycanes est d'assurer la structuration des fibrilles de collagène et d'élastine. L'acide hyaluronique est le glycosaminoglycane le plus abondant de la peau. Il est le constituant majeur du derme et est également présent autour des kératinocytes dans l'épiderme. Les GAGs sont des molécules très hygroscopiques qui ont la propriété de retenir jusqu'à mille fois leur poids en eau (Silbert JE. Proteoglycans and glycosaminoglycans. In: Goldsmith LA, ed. Biochemistry and Physiology of the Skin. New York, NY: Oxford University; 1983: 448-461). Grâce à ces propriétés exceptionnelles, les complexes de glycosaminoglycanes et de collagène sont des acteurs majeurs du stockage de l'eau dans les matrices extracellulaires.

Les pertes cutanées en eau peuvent avoir plusieurs origines, héréditaires, acquises ou liées à l'environnement. Dans un environnement très sec, les pertes en eau par évaporation à partir du *stratum corneum* sont significatives et peuvent excéder le taux de remplacement par diffusion transcellulaire.

Au cours du vieillissement cutané, la peau devient sèche. Ainsi, il est très souvent constaté chez les sujets âgés, et notamment de plus de 50 ans, la manifestation d'une xérose ou d'une sécheresse des muqueuses liée à une moindre sécrétion de sébum, à des modifications hormonales ou à des ralentissements du flux hydrique à travers l'épiderme. La peau est alors le siège de démangeaisons et de tiraillements, deux symptômes caractéristiques d'une peau sèche. Parmi les pathologies acquises se traduisant par une sécheresse de la peau, on peut citer la xérose induite par la photo-chimiothérapie et l'eczéma. Parmi les pathologies acquises provoquant une sécheresse de la bouche, ou xérostomie, on peut citer le syndrome de Sjogren ou la radiothérapie du cou. Enfin, parmi les pathologies impliquant une sécheresse des muqueuses, on peut citer les sécheresses oculaires ou vaginales.

Une première alternative de traitement des peaux sèches consiste à administrer par voie topique des produits destinés à restaurer la barrière cutanée, ou des agents filmogènes destinés à retenir l'eau. Toutefois, ces produits ont une action superficielle qui ne corrige pas les défauts biologiques d'une peau souffrant de déshydratation chronique. On peut également citer les brevets FR 2 801 504 et FR 2 874 502 qui décrivent l'utilisation d'extrait de plantes pour stimuler l'activité des aquaporines et pour améliorer l'hydratation de la peau. Malgré tout, ces ingrédients actifs ne permettent pas d'obtenir une amélioration de l'hydratation constitutive de tous les compartiments de la peau. Des extraits peptidiques de pois ont été décrits pour leur effet pigmentant (FR 2 904 556) ou leur effet desquamant (JP 09025225), mais ne décrivaient pas d'effet bénéfique sur l'hydratation de la peau. EP 2033617 concerne des compositions cosmétiques comprenant des protéines. Les inventeurs ont maintenant montré que l'utilisation d'un hydrolysat peptidique de pois *(Pisum sativum L.),* en tant qu'agent actif capable d'activer l'expression des aquaporines, des glycosaminoglycanes et de la filaggrine, procure une amélioration globale de l'hydratation constitutive de la peau.

### Exposé de l'invention

La présente invention a pour principal objectif une nouvelle utilisation d'un hydrolysat peptidique de pois (*Pisum sativum* L.) selon les revendications en tant qu'agent actif hydratant de la peau.

On entend par peau, l'ensemble des tissus de recouvrement constituant la peau et les muqueuses, y compris le cuir chevelu.

Il est bien évident que l'invention s'adresse aux mammifères en général, et plus particulièrement aux êtres humains.

Les inventeurs ont en effet mis en évidence des activités biologiques utiles pour améliorer l'hydratation constitutive de la peau, d'un hydrolysat peptidique de pois.

Les activités biologiques utiles caractéristiques selon l'invention sont définies *in vitro* par la capacité de l'hydrolysat de pois à augmenter l'expression des aquaporines, des glycosaminoglycanes et de la filaggrine.

On entend par «agent actif capable d'augmenter l'expression des aquaporines, des glycosaminoglycanes et de la filaggrine », toute substance capable d'augmenter la synthèse protéique de ces composés par modulation directe ou indirecte de l'expression génique ou par d'autres processus biologiques tels que l'assemblage post-traductionnel, ou la stabilisation des protéines.

Préférentiellement, selon la présente invention, l'aquaporine sera l'aquaporine 3, ou AQP3, présente dans les membranes des kératinocytes.

L'invention concerne plus particulièrement l'utilisation cosmétique d'un hydrolysat peptidique de pois *(Pisum sativum* L.) pour améliorer l'hydratation constitutive de la peau, grâce à son action positive sur les flux hydriques et l'augmentation de la capacité de stockage de l'eau dans la couche cornée, les couches basales de l'épiderme et le derme.

On entend par « hydratation constitutive » la teneur en eau liée de l'ensemble des compartiments de la peau, c'est-à-dire le derme, les couches basales de l'épiderme (ou épiderme prolifératif) et la couche cornée.

On entend par « hydrolysat peptidique », un mélange de composés majoritairement représentés par des peptides.

On entend par « application topique », le fait d'appliquer ou d'étaler l'agent actif selon l'invention, ou une composition le contenant, à la surface de la peau ou d'une muqueuse.

On entend par « physiologiquement acceptable », que l'agent actif selon l'invention, ou une composition le contenant, est approprié pour entrer en contact avec la peau ou une muqueuse sans provoquer de réactions de toxicité ou d'intolérance.

L'agent actif selon l'invention peut être obtenu par extraction de protéines d'origine végétale, suivie d'une hydrolyse contrôlée qui libère des fragments peptidiques biologiquement actifs.

De très nombreuses protéines trouvées dans les plantes sont susceptibles de contenir des fragments peptidiques biologiquement actifs au sein de leur structure. L'hydrolyse ménagée permet de dégager ces fragments peptidiques. Il est possible, mais non nécessaire pour réaliser l'invention, d'extraire soit les protéines concernées d'abord et de les hydrolyser ensuite, soit d'effectuer l'hydrolyse d'abord sur un extrait brut et de purifier les fragments peptidiques ensuite. Il est également possible d'utiliser certains extraits hydrolysés sans en purifier les fragments peptidiques correspondant aux peptides biologiquement actifs selon l'invention, mais en s'assurant toutefois de la présence desdits fragments par des moyens analytiques appropriés.

Pour réaliser l'extraction, on peut utiliser la plante entière, ou une partie spécifique de la plante (feuille, grain, etc.)

Plus particulièrement selon l'invention on utilise des graines de plante de la famille des Fabacées (légumineuses), de l'espèce des pois *Pisum sativum L.* Le terme pois désigne aussi la graine, elle-même riche en protéines (25 %).

Toute méthode d'extraction ou de purification connue de l'homme du métier peut être utilisée afin de préparer l'hydrolysat selon l'invention.

Dans une première étape, les graines sont broyées à l'aide d'un broyeur à plantes. La poudre ainsi obtenue peut ultérieurement être "délipidée" à l'aide d'un solvant organique classique (comme par exemple un alcool, l'hexane ou de l'acétone).

On réalise ensuite l'extraction des protéines suivant le procédé classique (Osborne, 1924) modifié ; le broyat de plante est mis en suspension dans une solution alcaline contenant un produit adsorbant de type polyvinylpolypyrrolidone (PVPP) insoluble (0,01 -20 %) ; en effet il a été observé que les opérations d'hydrolyses et de purifications ultérieures étaient facilitées par ce moyen. En particulier, la concentration en substances de type phénoliques, interagissant avec les protéines, se trouve nettement réduite.

La fraction soluble, contenant les protéines, les glucides et éventuellement des lipides, est recueillie après des étapes de centrifugation et de filtration. Cette solution brute est ensuite hydrolysée dans des conditions ménagées pour générer des peptides solubles. L'hydrolyse se définit comme étant une réaction chimique impliquant le clivage d'une molécule par de l'eau, cette réaction pouvant se faire en milieu neutre, acide ou basique. Selon l'invention, l'hydrolyse est réalisée par voie chimique et/ou de façon avantageuse par des enzymes protéolytiques. On peut alors citer l'utilisation des endoprotéases d'origine végétale (papaïne, bromelaïne, ficine) et de micro-organismes (Aspergillus, Rhizopus, Bacillus, etc.). Pour les mêmes raisons que précédemment, c'est-à-dire l'élimination des substances polyphénoliques, une quantité de polyvinylpolypyrrolidone est additionnée au milieu réactionnel lors de cette étape d'hydrolyse ménagée. Après désactivation thermique des enzymes et filtration, permettant d'éliminer les enzymes résiduelles et les polymères, le filtrat (solution) obtenu est encore purifié afin de sélectionner les composés de nature peptidique de bas poids moléculaires. Le fractionnement peut s'effectuer avantageusement par ultrafiltration et/ ou par une méthode de type chromatographique.

A cette étape, l'hydrolysat de pois est caractérisé par un poids sec de 70 à 80 g/kg, un taux de protéines de 55 à 65 g/l, un taux de sucres de 2 à 5 g/l et un taux de polyphénol de 1 à 3 g/l.

L' hydrolysat obtenu selon l'invention est analysé qualitativement et quantitativement, selon les techniques classiques bien connues de l'homme du métier, pour ses caractéristiques physico-chimiques et sa teneur en composés de nature protéique et peptidique. L'hydrolysat obtenu est composé de peptides de poids moléculaire inférieur à 5 kDa.

On procède ensuite à une phase de dilution dans de l'eau ou dans tout mélange de solvants contenant de l'eau. Ainsi, l'agent actif selon l'invention est avantageusement solubilisé dans un ou plusieurs solvants physiologiquement acceptables, tels que l'eau, le glycérol, l'éthanol, le propanediol, le butylène glycol, le dipropylène glycol, les diglycols éthoxylés ou propoxylés, les polyols cycliques ou tout mélange de ces solvants. L'agent actif dilué est ensuite stérilisé par filtration stérile.

Après cette étape, l'hydrolysat peptidique de pois est caractérisé par une teneur en composés peptidiques de 0,5 à 5,5 g/l. Cet hydrolysat peptidique correspond à l'agent actif selon l'invention.

Après cette étape de dilution, l'agent actif peut être encapsulé ou inclus dans un vecteur cosmétique ou pharmaceutique tels que les liposomes ou toute autre microcapsule utilisée dans le domaine de la cosmétique ou adsorbé sur des polymères organiques poudreux, des supports minéraux comme les talcs et bentonites.

L'invention a comme second aspect, l'utilisation d'une composition cosmétique comprenant une quantité efficace d'hydrolysat peptidique de pois selon l'invention, en tant qu'agent actif, dans un milieu physiologiquement acceptable, pour maintenir ou restaurer l'hydratation cutanée.

Selon cet aspect particulier de l'invention l'hydrolysat peptidique de pois est également utilisé en tant qu'agent actif dans une composition cosmétique pour lutter contre la sécheresse cutanée.

Selon un mode de réalisation avantageux de l'invention, l'agent actif selon l'invention est présent dans les compositions de l'invention en quantité efficace, soit à une concentration comprise entre 0,0001 % et 20 % environ, et préférentiellement à une concentration comprise entre 0,05 % et 5 % environ par rapport au poids total de la composition finale.

La composition utilisable selon l'invention pourra être appliquée par toute voie appropriée, notamment orale, parentérale ou topique, et la formulation des compositions sera adaptée par l'homme du métier, en particulier pour des compositions cosmétiques ou dermatologiques. Avantageusement, les compositions selon l'invention sont destinées à une administration par voie topique. Ces compositions doivent donc contenir un milieu physiologiquement acceptable, c'est-à-dire compatible avec la peau et les phanères, et couvrent toutes les formes cosmétiques ou dermatologiques.

Ces compositions pourront notamment se présenter sous forme d'une solution aqueuse, hydroalcoolique ou huileuse ; d'une émulsion huile-dans-eau, eau-dans-huile ou émulsions multiples ; elles peuvent aussi se présenter sous forme de crèmes, de suspensions, ou encore de poudres, adaptées à une application sur la peau, les muqueuses, les lèvres et/ou les phanères. Ces compositions peuvent être plus ou moins fluides et avoir l'aspect d'une crème, d'une lotion, d'un lait, d'un sérum, d'une pommade, d'un gel, d'une pâte ou d'une mousse. Elles peuvent aussi se présenter sous forme solide, comme un stick ou être appliquées sur la peau sous forme d'aérosol. Elles peuvent être utilisées comme produit de soin et/ou comme produit de maquillage de la peau.

L'ensemble de ces compositions comprennent, en outre, tout additif communément utilisé dans le domaine d'application envisagé ainsi que les adjuvants nécessaires à leur formulation, tels que des co-solvants (éthanol, glycérol, alcool benzylique, humectant...), des épaississants, des diluants, des émulsionnants, des anti-oxydants, des colorants, des filtres solaires, des pigments, des charges, des conservateurs, des parfums, des absorbeurs d'odeur, des huiles essentielles, des oligo-éléments, des acides gras essentiels, des tensioactifs, des polymères filmogènes, des filtres chimiques ou minéraux, des agents hydratants ou des eaux thermales etc. On peut par exemple citer des polymères hydrosolubles de type polymère naturel, tels que les polysaccharides, ou polypeptides, des dérivés cellulosiques de type méthylcellulose ou hydroxypropylcellulose, ou encore des polymères synthétiques, polaxamères, carbomères, siloxanes, PVA ou PVP et notamment les polymères vendus par la société ISP.

Dans tous les cas, l'homme de métier veillera à ce que ces adjuvants ainsi que leurs proportions soient choisis de telle manière à ne pas nuire aux propriétés avantageuses recherchées de la composition selon l'invention. Ces adjuvants peuvent, par exemple, être présents à des concentrations allant de 0,01 à 20 % du poids total de la composition. Lorsque la composition de l'invention est une émulsion, la phase grasse peut représenter de 5 à 80 % en poids et de préférence de 5 à 50 % en poids par rapport au poids total de la composition. Les émulsionnants et co-émulsionnants utilisés dans la composition seront choisis parmi ceux classiquement utilisés dans le domaine considéré. Par exemple, ils peuvent être utilisés en une proportion allant de 0,3 à 30 % en poids, par rapport au poids total de la composition.

Il est bien entendu que l'agent actif selon l'invention peut être utilisé seul ou bien en association avec d'autres ingrédients actifs.

Avantageusement, les compositions utilisables selon l'invention contiennent, en outre, au moins un autre agent actif destiné à favoriser l'action de l'agent actif selon l'invention. On peut citer, de manière non limitative, les classes d'ingrédients suivantes : d'autres agents actifs peptidiques, des extraits de végétaux, des agents cicatrisants, anti-âge, antirides, apaisants, anti-radicalaires, anti-UV, des agents stimulant la synthèse de macromolécules dermiques ou le métabolisme énergétique, des agents hydratants, antibactériens, antifongiques, anti-inflammatoires, anesthésiques, des agents modulant la différenciation, la pigmentation ou la dépigmentation cutanée, des agents stimulant la pousse des ongles ou des cheveux.

Préférentiellement, on utilisera un agent anti-radicalaire ou antioxydant, ou un agent stimulant la synthèse de macromolécules dermiques, ou encore un agent stimulant le métabolisme énergétique. Dans un mode plus particulier de réalisation, la composition selon l'invention comprendra, outre l'hydrolysat peptidique de pois,
- au moins un composé activateur du cytochrome c, et/ou ;
- au moins un composé hydratant, tel qu'un composé activateur des aquaporines et/ou ;
- au moins un composé activateur des sirtuines et/ou ;
- au moins un composé qui augmente l'adhésion cellulaire et/ou ;
- au moins un composé qui augmente la production de protéines matricielles telles que le collagène, fibronectine, laminine, glycosaminoglycanes et/ou;
- au moins un composé modulateur de l'activité du protéasome et/ou;
- au moins un composé modulateur du rythme circadien et/ou;
- au moins un composé modulateur des protéines HSP et/ou;
- au moins un composé qui augmente l'énergie cellulaire et/ou;
- au moins un composé modulant la pigmentation de la peau et/ou ;
- au moins un composé activateur du coenzyme Q10 et/ou ;
- au moins un composé améliorant la fonction barrière, tels que un composés activateur de la transglutaminase ou de la HMG-CoA réductase et/ou ;
- au moins un composé protecteur de la mitochondrie.

Lesdits composés ci-dessus peuvent être d'origine naturelle, comme des hydrolysats peptidiques de plantes, ou encore d'origine synthétique, comme des composés peptidiques.

L'invention a pour troisième objet un procédé de traitement cosmétique destiné à restaurer le déséquilibre hydrique qui se manifeste lors du vieillissement cutané, caractérisé en ce que l'on applique topiquement sur la peau à traiter une composition comprenant un hydrolysat peptidique de pois selon l'invention

L'invention a pour quatrième objet l'utilisation d'une quantité efficace d'hydrolysat peptidique de pois *(Pisum sativum* L.), en titre d'agent actif hydratant, pour la préparation d'une composition pharmaceutique destinée à prévenir ou traiter les sècheresses pathologiques de la peau.

Selon cette forme de l'invention, les compositions seront appropriées à une administration orale pour un usage pharmaceutique. Ainsi les compositions pourront notamment se présenter sous forme de comprimés, capsules, gélules, pâtes à mâcher, poudres à consommer telles quelles ou à mélanger extemporanément avec un liquide, sirops, gels, et toute autre forme connue de l'homme du métier. Ces compositions comprennent, en outre, tout additif communément utilisé dans le domaine d'application envisagé ainsi que les adjuvants nécessaires à leur formulation, tels que des solvants, des épaississants, des diluants, des antioxydants, des conservateurs, d'autres ingrédients actifs pharmaceutiques, des huiles essentielles, des vitamines, des acides gras essentiels, etc.

Des modes de réalisation particuliers de ce procédé de traitement cosmétique résultent également de la description précédente. D'autres avantages et caractéristiques de l'invention apparaîtront mieux à la lecture des exemples donnés à titre illustratif et non limitatif.

### Exemple 1 : Préparation d'un hydrolysat peptidique à partir de pois (Pisum sativum L.)

L'hydrolysat peptidique est obtenu à partir d'un extrait de plantes de l'espèce *Pisum sativum L.* Dans une première étape, 1 kg de pois décortiqués sont délipidés par l'action d'un solvant organique : l'hexane. La farine de pois ainsi obtenue est mise en solution dans 10 volumes d'eau en présence de 2 % de POLYCLAR® 10 (polyvinylpyrrolidone - PVPP - insoluble). Le mélange est ajusté à un pH compris entre 7 et 8 avec une solution aqueuse de soude 1 M.

Après ajustement du pH, sont rajoutés 2 % de flavourzym® dans le milieu réactionnel. L'hydrolyse est obtenue après 2 heures d'agitation à 50°C. On procède à la désactivation de l'enzyme en chauffant la solution à 80°C pendant 2 heures. Le mélange réactionnel ainsi obtenu correspond à un extrait de pois brut.

Le procédé de purification commence par des filtrations successives à l'aide de filtres-plaques Seitz-Orion de porosité décroissante (jusqu'à 0,2 µm) afin d'obtenir une solution brillante et limpide. A cette étape, l'hydrolysat de pois est caractérisé par un poids sec de 70-80 g/kg, un taux de protéines de 55-65 g/l, un taux de sucres de 2-5 g/l et un taux de polyphénol de 1-3 g/l.

La nature protéique de cet hydrolysat est mise en évidence par électrophorèse sur gel de polyacrylamide. Pour cette analyse, on utilise les gels NuPAGE® Bis-Tris Pre-cast (Invitrogen). L'hydrolysat peptidique de pois est chauffé à 70°C pendant 10 minutes dans des conditions réductrices dénaturantes dans un tampon de préparation d'échantillon NuPAGE® LDS. Une solution de NuPAGE® Antioxydant est ajoutée dans la cuve intérieure (cathode) pour éviter que les protéines réduites se ré-oxydent durant l'électrophorèse. La migration des protéines est réalisée à l'aide du tampon de migration NuPAGE® MES avec le standard SeeBlue Plus2 comme marqueur de poids moléculaire. La coloration des protéines est effectuée à l'aide de Bleu de Coomassie® R-250. Dans ces conditions, on observe 2 grandes familles de protéines : la 1ère famille correspond à des protéines de poids moléculaire de 25 à 20 kDa et la dernière famille à des protéines de poids moléculaires inférieurs à 5kDa.

Cette solution est alors purifiée en éliminant les protéines de poids moléculaires supérieurs à 5 kDa à l'aide de filtration à flux tangentiel.

Pour cela, l'hydrolysat de pois est pompé sous pression à travers un support Pellicon® équipé de cassette Pellicon®2 Biomax 30 kDa. Ce premier filtrat est récupéré pour être ensuite filtré à travers une autre cassette Pellicon® 2 Biomax 5 kDa. En fin de purification, on obtient un hydrolysat peptidique de pois jaune-beige, brillant et limpide. Il est caractérisé par un poids sec de 50 à 55 g/kg, une teneur en protéines de 50 à 52 g/l.

On procède ensuite à une phase de dilution dans un mélange eau-glycérol. L'agent actif dilué est ensuite stérilisé par filtration stérile. Après cette étape, l'hydrolysat peptidique de pois est caractérisé par une teneur en protéines d'environ 2,5 g/l.

### Exemple 2 : Mise en évidence de l'effet stimulant de l'extrait de pois selon l'exemple 1 sur l'expression d'aquaporines

Le but de cette étude est de déterminer l'influence de l'hydrolysat de pois selon l'exemple 1 sur l'expression de l'aquaporine 3 dans des kératinocytes humains normaux (KHN).

Protocole : Des KHN sont mis en culture dans des Labtek® jusqu'à une confluence de 80 % puis traités avec de l'hydrolysat de pois selon l'exemple 1 à 0,5 % ou 1 % pendant 24h.

L'immunomarquage est alors réalisé à l'aide d'un anticorps polyclonal de chèvre spécifique de l'aquaporine-3 (Tebu Santa Cruz, sc-9885) puis d'un anticorps secondaire, couplé à un marqueur fluorescent. Les cellules sont alors examinées au microscope à Epi-fluorescence (Nikon Eclipse E600 microscope).

Résultats : Les observations microscopiques montrent une fluorescence plus forte pour les cellules traitées avec l'hydrolysat de pois selon l'exemple 1 à 0,5 et 1 %.

Conclusions : L'hydrolysat de pois selon l'exemple 1 augmente l'expression de l'aquaporine 3 dans les kératinocytes humains.

### Exemple 3 : Etude de l'expression de la pro/filaggrine dans des biopsies de peau humaine, en présence de l'hydrolysat de pois selon l'exemple 1

Le but de cette étude est de déterminer l'influence de l'hydrolysat de pois selon l'exemple 1 sur la quantité de filaggrine et de profilaggrine présentes dans des biopsies de peau humaine.

Protocole : Des échantillons de peau humaine sont mis en culture à l'interface air/liquide. Les échantillons sont traités avec l'hydrolysat de pois selon l'exemple 1 à 0,5 % et 1 % pendant 24 h. Ces échantillons de peau sont ensuite fixés avec du formaldéhyde puis inclus dans de la paraffine. Des coupes de 4 µm sont alors réalisées. L'immunomarquage est effectué après démasquage des sites spécifiques par incubation dans de la pepsine. L'immunomarquage est alors réalisé à l'aide d'un anticorps monoclonal de souris spécifique de la filaggrine (Tebu Santa Cruz, sc-66192) puis d'un anticorps secondaire, couplé à un marqueur fluorescent. Les coupes de peau sont alors examinées au microscope à Epi-fluorescence (Nikon Eclipse E600 microscope).

Résultats : On observe un marquage plus intense de la pro/filaggrine sur les biopsies traitées avec l'hydrolysat de pois selon l'exemple 1, comparé aux biopsies de peau non traitées.

Conclusion: L'hydrolysat de pois selon l'exemple 1 a permis une augmentation de l'expression de la profilaggrine et de filaggrine dans la peau.

### Exemple 4 : Etude de l'expression des glycoaminoglycanes dans des biopsies de peau humaine, en présence de l'hydrolysat de pois selon l'exemple 1

Le but de cette étude est de déterminer l'influence de l'hydrolysat de pois selon l'exemple 1 sur la quantité de Glycosaminoglycanes (GAG) dans des biopsies de peau humaine.

Protocole : Des échantillons de peau humaine sont mis en culture à l'interface air/liquide. Les échantillons sont traités avec l'hydrolysat de pois selon l'exemple 1 à 0,5 % pendant 48 h. Ces échantillons de peau sont ensuite fixés avec du formaldéhyde puis inclus dans de la paraffine. Des coupes de 4 µm sont alors réalisées. La coloration des glycosaminoglycanes se fait à l'aide d'une solution de Fer Colloïdal, puis d'une réaction avec l'acide ferrocyanide-hydrochlorique, qui a la propriété de colorer les GAG en bleu.

Résultats : On observe une augmentation de l'expression des GAG sur les biopsies traitées avec l'extrait.

Conclusion: L'hydrolysat de pois selon l'exemple 1 a permis une augmentation de l'expression des glycosaminoglycanes dans la peau.

### Exemple 5 : Evaluation de l'effet protecteur de l'hydrolysat de pois selon l'exemple 1 vis-à-vis d'un assèchement cutané induit

Le but de cette étude est de déterminer l'effet protecteur de l'hydrolysat de pois selon l'exemple 1 vis-à-vis de cultures *ex vivo* soumises à un stress par un assèchement cutané induit.

Protocole : Des biopsies de peau humaine sont maintenues en culture *ex vivo* pendant 24 heures et traitées avec une solution à 0,5 ou 1 % de l'hydrolysat de pois selon l'exemple 1. Les biopsies sont alors soumises à une ventilation asséchante pendant 3h. Ces échantillons de peau sont ensuite fixés avec du formaldéhyde puis inclus dans de la paraffine. Des coupes de 4 µm sont alors réalisées. Les colorations histologiques hématoxyline-éosine (H&E) permettent d'évaluer la qualité des structures cutanées.

Résultats : Les biopsies contrôles ayant subi le stress asséchant montrent des signes de stress caractéristiques tels que la réduction de l'épaisseur du *stratum corneum,* la présence d'oedèmes ainsi qu'une désorganisation des keratinocytes basaux. Les coupes de peau asséchées et traitées avec l'hydrolysat de pois selon l'exemple 1 montrent une nette diminution des signes de stress cellulaire et une meilleure préservation des structures cutanées, comparées aux biopsies de peau non traitées. D'autre part, le stratum *stratum corneum* présente un aspect typique d'une meilleure hydratation, comparé aux biopsies de peau non traitées.

Conclusion : L'hydrolysat de pois selon l'exemple 1 protège efficacement la peau du stress induit par l'assèchement.

### Exemple 6 : Préparation de compositions

### 1 - Crème de jour

| ***Noms commerciaux*** | ***Noms INCI*** | ***% massique*** |
|---|---|---|
| ***PHASE A*** | | |
| MONTANOV 68 | Cetearyl Alcohol (and) Cetearyl Glucoside | 5,00 |
| HUILE DE JOJOBA | Simmondsia Chinensis (Jojoba) Seed Oil | 3,00 |
| HUILE DE VASELINE | Paraffinum Liquidum (Mineral Oil) | 2,00 |
| SQUALANE | Squalane | 3,00 |
| CERAPHYL 368 | Ethylhexyl palmitate | 4,00 |
| CERAPHYL 41 | C12-C15 Alkyl Lactate | 3,00 |
| RAPITHIX A-60 | Sodium polyacrylate (and) Hydrogenated Polydecene (and) Trideceth -6 | 0,30 |

| ***PHASE B*** | | |
|---|---|---|
| GLYCERINE | Glycerin | 5,00 |
| ALLANTOÏNE | Allantoin | 0,10 |
| EAU DEMINERALISEE | Aqua (Water) | qsp 100 |

| ***PHASEC*** | | |
|---|---|---|
| ROKONSAL MEP | Phenoxyethanol (and) Methylparaben (and) Ethylparaben (and) Propylparaben | 0,50 |

| ***PHASE D*** | | |
|---|---|---|
| AGENT ACTIF SELON EXEMPLE 1 | | 5% |

| ***PHASE E*** | | |
|---|---|---|
| PARFUM | Parfum (Fragrance) | qsp |

### Mode Opératoire :

Peser les ingrédients de la phase grasse et chauffer à 70°C sous agitation. Préparer la phase B et la chauffer à 70°C. Emulsionner la phase A dans la phase B. Ajouter la phase D vers 50°C sous agitation. En dessous de 40°C, additionner l'agent actif (Phase D). Parfumer et refroidir jusqu'à température ambiante.

### 2 - Crème hydratante W/O

| ***Noms commerciaux*** | ***Noms INCI*** | ***% massique*** |
|---|---|---|
| ***PHASE A*** | | |
| ARLACEL P 135 | PEG-30 Dipolyhydroxystearate Isononanoate | 2,00 |
| CERAPHYL 375 | Isostearyl Neopentanoate | 3,00 |
| PANALANE L-14E | Hydrogenated Polyisobutene | 3,00 |
| CERAPHYL ODS | Octyldodecyl Stearate | 9,00 |
| CERAPHYL 368 | Ethylhexyl Palmitate | 3,00 |

| ***PHASE B*** | | |
|---|---|---|
| EAU DEMINERALISEE | Aqua (Water) | qsp 100 |
| ATLAS G-2330 | Sorbeth-30 | 4,00 |
| SULFATE DE MAGNESIUM 7 H2O | Magnesium Sulfate | 0,70 |
| AGENT ACTIF SELON EXEMPLE 1 | | 2% |

| ***PHASE C*** | | |
|---|---|---|
| LIQUAPAR OPTIMA | Phenoxyethanol (and) Methylparaben (and) Isopropylparaben (and) Isobutylparaben (and) Butylparaben | 0,50 |

| ***PHASE D*** | | |
|---|---|---|
| PARFUM | Parfum (Fragrance) | qsp |

### Mode Opératoire :

Peser la phase A et chauffer à 75°C sous agitation. Préparer la phase B et la chauffer à 75°C. Emulsionner la phase B dans la phase A sous forte agitation rotor-stator.

Homogénéiser quelques minutes. Refroidir brutalement à l'aide d'un bain d'eau glacée sous vive agitation. Ajouter la phase C aux environs de 50°C et additionner le parfum (phase D) à 40°C. Poursuivre le refroidissement jusqu'à température ambiante.

### 3 - Lotion hydratante

| ***Noms commerciaux*** | ***Noms INCI*** | ***% massique*** |
|---|---|---|
| EAU DEMINERALISEE | Aqua (Water) | qsp 100 |
| GLYCERINE | Glycerin | 2,00 |
| PROPYLENE GLYCOL | Propylene Glycol | 2,00 |
| GLUCAM E-10 | Methyl Gluceth -10 | 1,00 |
| NEOSORB | Sorbitol | 5,00 |
| ALLANTOINE | Allantoin | 0,10 |
| ROKONSAL BSB | Benzoic Acid (and) Sorbic Acid (and) Benzyl Alcohol | 0,30 |
| AGENT ACTIF SELON EXEMPLE 1 | | 0,5 % |
| PARFUM hydrosoluble | Parfum (Fragrance) | qsp |

### Mode Opératoire :

Incorporer les ingrédients un à un à la quantité d'eau nécessaire et agiter jusqu'à parfaite dissolution. Réajuster le pH aux environs de 5,5 si nécessaire. Incorporer l'actif en fin de formulation. Parfumer avec un parfum hydrosoluble sous faible agitation.

## Revendications

1. Utilisation cosmétique non-thérapeutique d'un hydrolysat peptidique de pois (*Pisum sativum L.*), en tant qu'agent actif hydratant de la peau, comprenant entre 0,5 et 5,5 g/l de peptides ayant un poids moléculaire inférieur à 5 kDa et selon laquelle, pour la préparation dudit hydrolysat, des graines de pois sont broyées, le broyat de plante est mis en suspension dans une solution alcaline contenant un produit absorbant de type polyvinylpolypyrrolidone insoluble, la fraction soluble est recueillie après des étapes de centrifugation et de filtration, cette solution brute étant ensuite hydrolysée par des enzymes protéolytiques, une quantité de polyvinylpolypyrrolidone étant additionnée au milieu réactionnel lors de cette étape d'hydrolyse ménagée, puis, après désactivation thermique des enzymes et filtration, le filtrat obtenu est encore purifié afin de sélectionner les composés de nature peptidique de bas poids moléculaire, l'hydrolysat étant caractérisé, à cette étape, par un poids sec de 70 à 80 g/kg, un taux de protéines de 55 à 65 g/l, un taux de sucres de 2 à 5 g/l et un taux de polyphénol de 1 à 3 g/l, puis on procède à une phase de dilution dans de l'eau ou dans tout mélange de solvants contenant de l'eau tels que l'eau, le glycérol, l'éthanol, le propanediol, le butylène glycol, le dipropylène glycol, les diglycols éthoxylés ou propoxylés, les polyols cycliques ou tout mélange de ces solvants, l'agent actif étant ensuite stérilisé par filtration stérile, l'hydrolysat de pois étant caractérisé, après cette étape, par une teneur en composés peptidiques de 0,5 à 5,5 g/l.

2. Utilisation selon la revendication 1, **caractérisée en ce que** l'agent actif augmente l'expression des aquaporines et en particulier de l'aquaporine 3.

3. utilisation selon la revendication 1, **caractérisée en ce que** l'agent actif augmente l'expression des glycosaminoglycanes.

4. Utilisation selon la revendication 1, **caractérisée en ce que** l'agent actif augmente l'expression de la filaggrine.

5. Utilisation non-thérapeutique d'une composition cosmétique pour maintenir ou restaurer l'hydratation cutanée comprenant a) une quantité efficace dudit hydrolysat peptidique de pois tel que défini dans l'une des revendications précédentes, en tant qu'agent actif, et b) un milieu physiologiquement acceptable.

6. Utilisation d'une composition cosmétique selon la revendication 5 pour lutter contre la sécheresse cutanée.

7. Utilisation selon l'une des revendications 5 ou 6, **caractérisée en ce que** ledit hydrolysat peptidique de pois est utilisé à une concentration comprise entre 0,0001 % et 20 % du poids total de la composition.

8. Utilisation selon la revendication 7, **caractérisée en ce que** ledit hydrolysat peptidique de pois est utilisé à une concentration comprise entre 0,05 % et 5 % du poids total de la composition.

9. Utilisation selon l'une des revendications 5 à 8, **caractérisée en ce que** la composition se présente sous une forme adaptée à l'application par voie topique.

10. Utilisation selon l'une des revendications 5 à 9, **caractérisée en ce que** la composition contient en outre au moins un autre agent actif destiné à favoriser l'action dudit hydrolysat peptidique de pois, tel que défini dans l'une des revendications 1 à 5.

11. Utilisation selon la revendication 10, **caractérisée en ce que** ledit autre agent actif est un hydrolysat peptidique de plantes ou un composé peptidique de synthèse.

12. Procédé de traitement cosmétique non-thérapeutique destiné à restaurer le déséquilibre hydrique qui se manifeste lors du vieillissement cutané, **caractérisé en ce que** l'on applique topiquement sur la peau à traiter une composition telle que définie selon l'une quelconque des revendications 5 à 11.

13. Composition pharmaceutique comprenant une quantité efficace d'hydrolysat peptidique de pois (*Pisum sativum L*.), à titre d'agent actif hydratant, pour son utilisation dans la prévention ou le traitement des sécheresses pathologiques de la peau.

## Patentansprüche

1. Kosmetische, nicht therapeutische Verwendung eines Peptidhydrolysats aus Erbsen *(Pisum sativum L.)* als Befeuchtungswirkstoff der Haut, umfassend zwischen 0,5 und 5,5 g/l Peptide, aufweisend ein Molekulargewicht von weniger als 5 kDa, und gemäß der, zur Herstellung des Hydrolysats, Erbsenkerne gemahlen werden, das Mahlgut der Pflanze in einer alkalischen Lösung suspendiert wird, die ein absorbierendes Produkt vom Typ unlösliches Polyvinylpolypyrrolidon enthält, die lösliche Fraktion nach Schritten des Zentrifugierens und Filtrierens gesammelt wird, wobei diese Rohlösung dann durch proteolytische Enzyme hydrolysiert wird, wobei eine Menge von Polyvinylpolypyrrolidon dem Reaktionsmedium bei diesem Schritt des schonenden Hydrolysierens zugegeben wird, dann, nach der thermischen Deaktivierung der Enzyme und Filtration das erhaltene Filtrat weiter gereinigt wird, um die Verbindungen peptidischer Art mit niedrigem Molekulargewicht auszuwählen, wobei das Hydrolysat in diesem Schritt durch ein Trockengewicht von 70 bis 80 g/kg, einen Eiweißgehalt von 55 bis 65 g/l, einen Zuckergehalt von 2 bis 5 g/l und einen Polyphenolgehalt von 1 bis 3 g/l gekennzeichnet ist, dann zu einem Schritt des Verdünnens in Wasser oder in jeder Mischung von Lösemitteln, das Wasser enthält, wie z. B. Wasser, Glycerol, Ethanol, Propandiol, Butylendiol, Dipropylenglycol, ethoxylierte oder propoxylierte Diglycole, cyclische Polyole oder jede Mischung dieser Lösemittel, übergegangen wird, wobei der Wirkstoff dann durch steriles Filtrieren sterilisiert wird, wobei das Hydrolysat aus Erbsen nach diesem Schritt durch einen Gehalt an peptidischen Verbindungen von 0,5 bis 5,5 g/l gekennzeichnet ist.

2. Verwendung nach Anspruch 1, **dadurch gekennzeichnet, dass** der Wirkstoff die Expression der Aquaporine und insbesondere von Aquaporine 3 erhöht.

3. Verwendung nach Anspruch 1, **dadurch gekennzeichnet, dass** der Wirkstoff die Expression der Glycosaminoglykane erhöht.

4. Verwendung nach Anspruch 1, **dadurch gekennzeichnet, dass** der Wirkstoff die Expression des Filaggrins erhöht.

5. Nicht therapeutische Verwendung einer kosmetischen Zusammensetzung, um die Hautfeuchtigkeit beizubehalten oder wiederherzustellen, umfassend a) eine wirksame Menge des Peptidhydrolysats aus Erbsen, wie in einem der vorhergehenden Ansprüche definiert, als Wirkstoff, und b) ein physiologisch annehmbares Medium.

6. Verwendung einer kosmetischen Zusammensetzung nach Anspruch 5, um gegen die Trockenheit der Haut zu kämpfen.

7. Verwendung nach einem der Ansprüche 5 oder 6, **dadurch gekennzeichnet, dass** das Peptidhydrolysat aus Erbsen in einer Konzentration verwendet wird, die im Bereich zwischen 0,0001 % und 20 % des Gesamtgewichts der Zusammensetzung liegt.

8. Verwendung nach Anspruch 7, **dadurch gekennzeichnet, dass** das Peptidhydrolysat aus Erbsen in einer Konzentration verwendet wird, die im Bereich zwischen 0,05 % und 5 % des Gesamtgewichts der Zusammensetzung liegt.

9. Verwendung nach einem der Ansprüche 5 bis 8, **dadurch gekennzeichnet, dass** die Zusammensetzung eine Form aufweist, die an die Verabreichung auf topischem Weg angepasst ist.

10. Verwendung nach einem der Ansprüche 5 bis 9, **dadurch gekennzeichnet, dass** die Zusammensetzung außerdem mindestens einen weiteren Wirkstoff enthält, der ausgelegt ist, um die Wirkung des Peptidhydrolysats aus Erbsen zu fördern, wie in einem der Ansprüche 1 bis 5 definiert.

11. Verwendung nach Anspruch 10, **dadurch gekennzeichnet, dass** der andere Wirkstoff ein Peptidhydrolysat aus Pflanzen oder eine Synthese-Peptidverbindung ist.

12. Verfahren zu nicht therapeutischen kosmetischen Behandlung, die ausgelegt ist, um die Feuchtigkeitsstörung zu beheben, die sich bei der Alterung der Haut einstellt, **dadurch gekennzeichnet, dass** topisch auf die zu behandelnde Haut eine Zusammensetzung angewendet wird, wie in einem beliebigen der Ansprüche 5 bis 11 definiert.

13. Pharmazeutische Zusammensetzung, umfassend eine wirksame Menge Peptidhydrolysat aus Erbsen *(Pisum sativum* L.) als Befeuchtungswirkstoff für ihre Verwendung bei der Prävention oder der Behandlung von pathologischen Trockenheiten der Haut.

## Claims

1. Non-therapeutic cosmetic use of a peptide hydrolysate of pea *(Pisum sativum L.),* as an active agent that hydrates the skin, comprising between 0.5 and 5.5g/l of peptides having a molecular weight of less than 5kDa and wherein, for the preparation of said hydrolysate, pea seeds are ground, the ground plant material is placed in suspension in an alkaline solution containing an absorbent product such as insoluble polyvinylpolypyrrolidone, the soluble fraction is recovered after steps of centrifugation and filtration, this crude solution then being hydrolysed by proteolytic enzymes, a quantity of polyvinylpolypyrrolidone being added to the reaction medium during this step of mild hydrolysis, and then, after thermal deactivation of the enzymes and filtration, the filtrate obtained is again purified in order to select the compounds of a peptide nature having a low molecular weight, the hydrolysate being characterised, at this stage, by a dry weight of 70 to 80g/kg, a protein concentration of 55 to 65g/l, a sugar concentration of 2 to 5g/l and a polyphenol concentration of 1 to 3g/l, then there is a phase of dilution in water or in any mixture of solvents containing water such as water, glycerol, ethanol, propanediol, butylene glycol, dipropylene glycol, the ethoxylated or propoxylated diglycols, the cyclic polyols or any mixture of these solvents, the active agent then being sterilised via sterile filtration, the hydrolysate of pea being characterised, after this step, by a concentration of peptide compounds of 0.5 to 5.5g/l.

2. Use according to claim 1, **characterised in that** the active agent increases the expression of the aquaporins and in particular of aquaporin 3.

3. Use according to claim 1, **characterised in that** the active agent increases the expression of the glycosaminoglycans.

4. Use according to claim 1, **characterised in that** the active agent increases the expression of filaggrin.

5. Non-therapeutic use of a cosmetic composition for maintaining or restoring the hydration of the skin, comprising a) an effective quantity of said peptide hydrolysate of pea as defined in one of the previous claims, as an active agent, and b) a physiologically acceptable medium.

6. Use of a cosmetic composition according to claim 5 in order to combat dryness of the skin.

7. Use according to one of claims 5 and 6, **characterised in that** said peptide hydrolysate of pea is used at a concentration between 0.0001% and 20% of the total weight of the composition.

8. Use according to claim 7, **characterised in that** said peptide hydrolysate of pea is used at a concentration between 0.05% and 5% of the total weight of the composition.

9. Use according to one of claims 5 to 8, **characterised in that** the composition is in a form suitable for topical use.

10. Use according to one of claims 5 to 9, **characterised in that** the composition further contains at least one other active agent intended to promote the action of said peptide hydrolysate of pea, as defined in one of claims 1 to 5.

11. Use according to claim 10, **characterised in that** said other active agent is a peptide hydrolysate of plants or a synthetic peptide compound.

12. Method for non-therapeutic cosmetic treatment intended to restore the water imbalance that manifests itself during aging of the skin, **characterised in that** a composition as defined according to any one of claims 5 to 11 is applied topically onto the skin to be treated.

13. Pharmaceutical composition comprising an effective quantity of peptide hydrolysate of pea *(Pisum sativum L.),* as a hydrating active agent, for its use in the prevention or the treatment of pathological dryness of the skin.
